# DEMANDE DE BREVET EUROPEEN

(11) **EP 1 604 586 A1**
(43) Date de publication de la demande: **14.12.2005**
(21) Numéro de dépôt: 05300467.7
(22) Date de dépôt: 08.06.2005
(51) Int. Cl.: A45D 34/04

(54) **Dispositif d'application d'un produit, notamment cosmétique**

(30) Priorité: 08.06.2004 FR 0451133
(71) Demandeur: L'OREAL, 75008 Paris (FR)
(72) Inventeur: GUERET, Jean-Louis, 75016, PARIS (FR)
(74) Mandataire: Le Coupanec, Pascale A.M.P.

(57) **Abrégé**

La présente invention concerne un dispositif (1) d'application d'un produit (P), comportant :
- un élément d'application (22) ayant une surface (24) apte à se charger en produit (P),
- un excitateur électrique (29) comportant au moins deux électrodes (30) en contact permanent avec l'élément d'application (22) et permettant de faire circuler un courant électrique entre ces deux électrodes (30).

## Description

La présente invention a pour objet un dispositif d'application d'un produit, notamment cosmétique, y compris de soin.

Par « produit cosmétique », on entend au sens de la présente invention un produit tel que défini dans la Directive 93/35/CEE du 14 juin 1993 modifiant la Directive 76/768/CEE.

On connaît par la demande de brevet US 2003/0129016 un dispositif comportant un récipient contenant le produit à appliquer et une structure poreuse défmissant une surface servant à l'application, alimentée par le produit.

Il existe un besoin pour améliorer encore les dispositifs utilisés pour l'application d'un produit.

La présente invention vise notamment à faciliter ou renforcer l'action du produit sur la région du corps ou du visage traitée et/ou créer de nouveaux effets à l'application.

L'invention a ainsi pour objet, selon un premier de ses aspects, un dispositif d'application d'un produit, comportant :
- un élément d'application ayant une surface apte à se charger en produit,
- un excitateur électrique comportant au moins deux électrodes en contact permanent avec l'élément d'application et permettant de faire circuler un courant électrique entre ces deux électrodes.

On entend par "contact permanent" le fait que les électrodes et l'élément d'application sont maintenus en contact au moins lorsque le dispositif est en état de fonctionnement. Cela n'exclut pas que l'élément d'application, le cas échéant, puisse être séparé des électrodes, notamment pour être remplacé par un autre, entre deux utilisations du dispositif

L'invention offre de nombreux avantages.

Tout d'abord, l'élément d'application peut être rechargé en produit de manière relativement simple, par exemple par mise en contact d'une surface de cet élément d'application avec du produit.

De plus, le dispositif d'application selon l'invention peut être manipulé de manière relativement aisée.

La circulation du courant entre les électrodes peut favoriser par exemple l'ouverture et la dilatation des pores de la peau et faciliter l'action et/ou la pénétration d'au moins un actif du produit dans la peau.

L'invention peut également permettre, le cas échéant, d'activer la microcirculation sanguine, d'améliorer le tonus musculaire ou la capacité de cicatrisation de la peau.

Dans un exemple de mise en oeuvre de l'invention, l'élément d'application est électriquement conducteur à sec. Ainsi, du courant électrique peut circuler entre les deux électrodes indépendamment du fait que le produit à appliquer soit électriquement conducteur ou non. Cela peut permettre, le cas échéant, de chauffer localement l'élément d'application.

L'élément d'application peut comporter par exemple un matériau intrinsèquement peu conducteur, par exemple une matière plastique, renfermant une charge électriquement conductrice. Cette charge peut notamment contenir des particules électriquement conductrices, par exemple métalliques, choisies notamment dans la liste suivante : particules de cuivre, d'aluminium, d'argent et de graphite.

Dans un autre exemple de mise en oeuvre de l'invention, l'élément d'application est électriquement isolant à sec, auquel cas la circulation d'un courant électrique entre les électrodes peut s'établir seulement lorsque l'élément d'application est imprégné d'un produit électriquement conducteur.

Afin d'accroître l'intensité du courant électrique circulant entre les électrodes, l'élément d'application peut être électriquement conducteur à sec et le produit peut également être électriquement conducteur.

L'élément d'application peut en outre comporter, le cas échéant, des particules magnétiques, éventuellement enrobées, afm de créer un champ magnétique.

Lors de l'utilisation, les effets du champ magnétique liés aux particules magnétiques peuvent alors se cumuler à ceux liés au passage du courant électrique.

Dans un exemple de mise en oeuvre de l'invention, l'élément d'application est poreux et/ou élastiquement déformable, l'élément d'application pouvant notamment comporter au moins l'un des éléments choisis dans la liste suivante : une mousse, un fritté, un feutre.

L'élément d'application peut être capable de retenir du produit par capillarité et comporter par exemple une touffe de poils ou une pièce souple, notamment en silicone ou élastomère, adaptée à retenir du produit par capillarité.

L'élément d'application peut comporter une structure multicouche avec par exemple au moins une couche conductrice prise en sandwich entre deux autres couches. L'élément d'application peut comporter par exemple, successivement de l'extérieur vers l'intérieur, une couche extérieure en feutre, une couche conductrice, par exemple en métal, notamment en aluminium, et une couche en mousse, l'une au moins des électrodes pouvant être reliée à la couche conductrice.

L'élément d'application peut être hydrophile ou non.

L'une au moins des électrodes, notamment une portion terminale de celle-ci, peut être au moins partiellement noyée, notamment de manière permanente, dans l'élément d'application. Une telle caractéristique s'avère avantageuse notamment lorsque l'électrode en question est métallique, afin d'éviter un contact direct avec la peau.

Dans un exemple de mise en oeuvre de l'invention, l'élément d'application comportant une surface destinée à venir au contact de la zone à traiter, l'une au moins des électrodes ne présente aucun contact avec cette surface de l'élément d'application.

Dans un autre exemple de mise en oeuvre de l'invention, les électrodes peuvent épouser la forme extérieure de l'élément d'application et présenter une surface arrondie ou plate, par exemple.

L'une au moins des électrodes peut par exemple venir à affleurement de la surface extérieure de l'élément d'application, cette électrode comportant par exemple une tête arrondie de façon à ne pas blesser l'utilisateur.

Lorsque le dispositif comporte un élément de préhension, l'une au moins des électrodes peut être rigidement fixée à l'élément de préhension.

Dans un exemple de mise en oeuvre de l'invention, le dispositif comporte un compartiment pour recevoir une source électrique permettant d'alimenter électriquement les électrodes, par exemple au moins une pile électrique, accumulateur ou condensateur.

Dans un exemple de mise en oeuvre de l'invention, lors de l'utilisation, la tension aux bornes des électrodes est identique à la tension aux bornes de la source électrique. En variante, l'excitateur électrique comporte des moyens de raccordement à un adaptateur électrique, notamment un transformateur, branché sur le secteur.

La tension aux bornes des électrodes peut être inférieure à 30 volts, étant comprise entre 1 volt et 9 volts, de préférence.

L'excitateur électrique peut comporter un témoin lumineux qui s'allume lorsque le courant circule entre les électrodes, ce témoin pouvant comporter par exemple une diode électroluminescente, éventuellement bicolore.

L'excitateur électrique peut comporter un organe de réglage de la puissance, par exemple de la tension et/ou du courant maximal, notamment un potentiomètre ou un contacteur à plusieurs positions.

L'excitateur électrique peut comporter un interrupteur, avec par exemple un bouton, pour contrôler l'alimentation électrique des électrodes et notamment mettre en marche ou non le dispositif.

Le dispositif d'application peut comporter un réservoir contenant le produit et agencé pour alimenter l'élément d'application en produit.

Le produit présent dans le réservoir peut être électriquement conducteur et contenir par exemple des sels minéraux. Le produit peut être un produit cosmétique, par exemple destiné à effectuer un traitement capillaire, ou dermatologique. Le produit peut être apte à former un film sur la zone à traiter.

Dans un exemple de mise en oeuvre de l'invention, le dispositif comporte, d'une part, un récipient avec un réservoir de produit et, d'autre part, un applicateur comprenant un élément de préhension solidaire de l'élément d'application, l'applicateur étant agencé pour être mis en place sur le récipient de manière amovible, pour charger l'élément d'application en produit.

Avantageusement, l'applicateur comporte un compartiment pour loger une source électrique, ce compartiment pouvant être réalisé dans l'élément de préhension par exemple.

Le récipient peut comporter un logement pour recevoir l'élément d'application lorsque l'applicateur est monté sur le récipient. Ce logement peut comporter une paroi de fond agencée pour limiter le passage de produit du réservoir vers l'élément d'application, cette paroi comportant par exemple un ou plusieurs orifices.

Lorsque l'élément d'application est disposé dans le logement du récipient en vue du stockage, l'élément d'application peut être comprimé ou non.

Dans un autre exemple de mise en oeuvre de l'invention, le dispositif comporte un récipient contenant le produit à appliquer et l'élément d'application est fixé à demeure sur le récipient. Ce dernier peut comporter un compartiment pour loger une source électrique. En variante, le dispositif comporte un boîtier distinct du récipient, agencé pour loger une source électrique et recevoir au moins partiellement le récipient, de manière amovible. Ce boîtier comporte avantageusement des premiers contacteurs électriques agencés pour coopérer avec des deuxièmes contacteurs électriques solidaires du récipient, pour alimenter électriquement les électrodes.

Lorsque le dispositif comporte un compartiment pour loger une source électrique, ce compartiment peut être pourvu d'un organe de rappel élastique, disposé notamment dans le fond du compartiment, agencé pour déplacer la source électrique d'une première position dans laquelle la source électrique alimente les électrodes, vers une deuxième position dans laquelle la source électrique est déconnectée des électrodes. Pour provoquer l'alimentation électrique des électrodes, l'utilisateur exerce une force sur la source électrique contre l'effort de rappel de l'organe de rappel élastique et amène la source électrique dans la première position. Le dispositif peut ainsi être dépourvu d'interrupteur distinct de la source électrique et sa construction est simplifiée.

L'organe de rappel élastique peut comporter un ressort, notamment un ressort hélicoïdal.

Dans un exemple de mise en oeuvre de l'invention, le récipient comporte un logement pour recevoir l'élément d'application, de manière amovible ou non, ce logement étant en communication avec une réserve de produit.

L'invention a encore pour objet, selon un autre de ses aspects, un applicateur comportant :
- un élément de préhension,
- un élément d'application solidaire de l'élément de préhension et ayant une surface apte à se charger en produit,
- un excitateur électrique comportant au moins deux électrodes en contact permanent avec l'élément d'application et permettant de faire circuler un courant électrique entre ces deux électrodes,
l'applicateur étant dépourvu de réservoir de produit associé à l'élément d'application, permettant de l'alimenter en produit.

L'applicateur peut comporter un compartiment pour loger une source électrique.

L'invention a encore pour objet, selon un autre de ses aspects, un procédé de traitement, notamment cosmétique, par exemple d'une zone de la peau, comportant les étapes suivantes :
- fournir un dispositif d'application d'un produit, comportant:

- un élément d'application ayant une surface apte à se charger en produit,
- un excitateur électrique comportant au moins deux électrodes en contact permanent avec l'élément d'application pour faire circuler un courant électrique entre ces deux électrodes,
- charger l'élément d'application avec du produit,
- appliquer du produit sur une zone à traiter à l'aide de l'élément d'application.

Dans un exemple de mise en oeuvre de l'invention, on fait circuler un courant électrique entre les deux électrodes simultanément à l'application de produit sur la zone à traiter.

Le courant électrique peut, le cas échéant, être appliqué de manière séquentielle, soit manuellement en manipulant par exemple un interrupteur du dispositif, soit de manière automatique grâce à un circuit électronique adapté.

En variante, le produit peut être appliqué à l'aide de l'élément d'application sans faire circuler de courant électrique entre les électrodes puis, le cas échéant, un courant électrique est appliqué entre les électrodes sensiblement sans appliquer une quantité supplémentaire de produit, l'élément d'application étant en contact avec la zone déjà recouverte de produit.

Lorsque l'élément d'application est électriquement isolant à sec, le procédé comporte avantageusement les étapes suivantes :
- charger l'élément d'application avec un produit électriquement conducteur,
- faire circuler un courant électrique entre les électrodes.

L'invention a encore pour objet, selon un autre de ses aspects, un dispositif d'application d'un produit, comportant :
- un élément de préhension,
- un élément d'application ayant une surface apte à se charger en produit, l'élément d'application étant solidaire de l'élément de préhension au moins lors de l'application du produit,
- un excitateur électrique comportant au moins deux électrodes au contact de l'élément d'application et permettant de faire circuler un courant électrique entre ces deux électrodes.

L'invention a encore pour objet, selon un autre de ses aspects, un dispositif d'application d'un produit, comportant :
- un élément d'application ayant une surface apte à se charger en produit,
- un excitateur électrique comportant au moins deux électrodes au contact de l'élément d'application et permettant de faire circuler un courant électrique entre ces deux électrodes à volonté,
- un élément de préhension avec un compartiment pour loger une source électrique permettant d'alimenter électriquement l'excitateur électrique, le compartiment étant pourvu d'un organe de rappel élastique agencé pour déplacer la source électrique d'une première position dans laquelle la source électrique alimente les électrodes, vers une deuxième position dans laquelle la source électrique est déconnectée des électrodes.

L'invention a encore pour objet, selon un autre de ses aspects, un dispositif d'application d'un produit, comportant :
- un élément d'application ayant une surface apte à se charger en produit, l'élément d'application comportant au moins l'un des éléments choisis dans la liste suivante : une mousse, un fritté, un feutre, une pièce en élastomère, notamment en silicone, une touffe de poils,
- un excitateur électrique comportant au moins deux électrodes au contact de l'élément d'application et permettant de faire circuler un courant électrique entre ses deux électrodes.

La présente invention pourra être mieux comprise à la lecture de la description détaillée qui va suivre, d'exemples de mise en oeuvre non limitatifs de l'invention, et à l'examen du dessin annexé, sur lequel :
- la figure 1 représente, schématiquement et partiellement, en coupe axiale, un dispositif d'application conforme à l'invention,
- la figure 2 représente, schématiquement et partiellement, en coupe axiale, un applicateur conforme à une variante de mise en oeuvre de l'invention,
- la figure 3 est une vue schématique et partielle, en coupe axiale, d'un dispositif d'application conforme à un autre exemple de mise en oeuvre de l'invention,
- la figure 4 représente, schématiquement et partiellement, un récipient d'un dispositif d'application conforme à un autre exemple de mise en oeuvre de l'invention,
- la figure 5 représente, schématiquement et partiellement, le dispositif d'application de la figure 4, avec le boîtier porteur de l'alimentation électrique, dans lequel est disposé l'applicateur, et
- les figures 6 et 7 représentent schématiquement et partiellement des variantes d'éléments d'application.

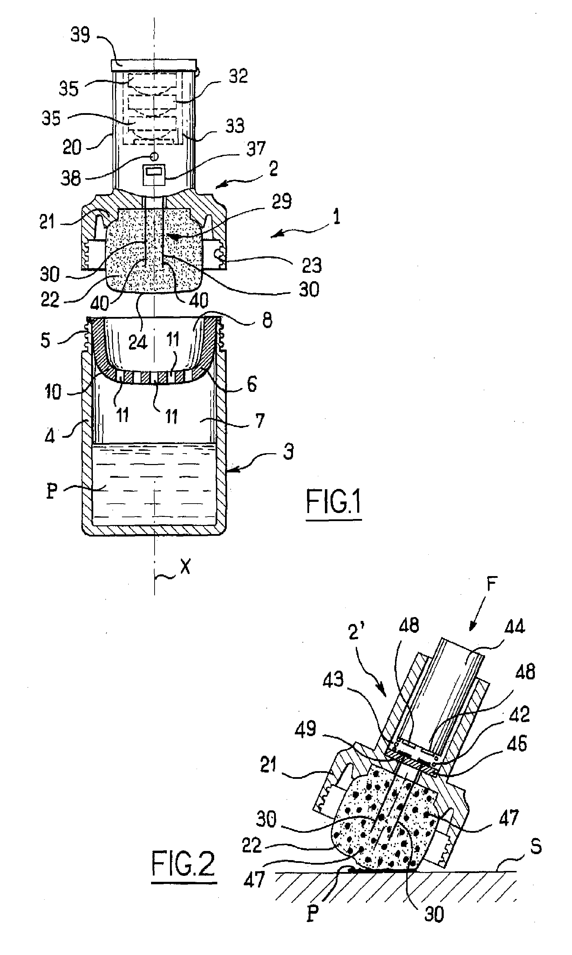

On a représenté sur la figure 1 un dispositif d'application 1 conforme à l'invention, comportant un applicateur 2 et un récipient 3 pouvant être séparé de l'applicateur 2.

Le récipient 3 comporte un corps 4, d'axe X, formant un réservoir 7 pour un produit P.

Dans l'exemple considéré, le produit P est un produit cosmétique ou dermatologique destiné à être appliqué sur la peau. En variante, le produit P peut être un produit capillaire. Le produit P est par exemple un liquide ou une poudre. Le produit P peut contenir au moins un actif cosmétique ou dermatologique, notamment au moins un composé choisi dans la liste suivante : métaux et leurs alliages, cobalt, baryum, chrome, aluminium, argent, cuivre, titane, bronze, manganèse, oxydes métalliques, oxydes de fer, notamment ferrite, terres rares, silicates, sulfates, notamment sulfate de baryum, carbonates, notamment carbonate de calcium, composés non ferreux, notamment soufre, magnésium, calcium, bore, potassium, carbone, oligo-éléments, sel marin, sels gemmes, argile, stéatite, algues et planctons et leurs extraits, racines, réglisse, gingembre, cires huileuses, protéines, hormones, collagènes, aluns, notamment pierre d'alun, glucose, vitamines, notamment vitamine C, vitamine A, vitamine F, vitamine B, vitamine E, extraits végétaux, glycérine, laponite, tensioactifs, collagène, acides, notamment acide salicylique, acide tio, caféine, huiles essentielles aromatiques, colorants, anti-oxydants, anti-radicaux libres, hydroabsorbants, hydratants, dépigmentants, liporégulateurs, anti-acnéique, anti-séborrhéiques, agents anti-vieillissement, adoucissants, anti-rides, kératolitiques, anti-inflammatoires, rafraîchissants, cicatrisants, protecteurs vasculaires, anti-bactériens, antifongiques, anti-perspirants, déodorants, conditionneurs de la peau, insensibilisants, immunomodulateurs, nourrissants.

Le corps 4 est pourvu d'un col 5 fileté extérieurement et dans lequel est disposé un réducteur d'écoulement 6. Celui-ci forme un logement 8, délimité inférieurement par une paroi de fond 10 réalisée sous la forme d'une grille avec une pluralité d'orifices 11. En variante, la paroi de fond 10 ne peut comporter qu'un unique orifice.

L'applicateur 2 comporte un manche 20 d'axe longitudinal par exemple confondu avec l'axe X.

Dans l'exemple considéré, le manche 20 se raccorde à une portion de support 21 de l'élément d'application 22 et à une jupe filetée 23 permettant de visser l'applicateur 2 sur le col 5 du récipient 3.

En variante, l'applicateur 2 et le récipient 3 peuvent être agencés pour permettre une mise en place par encliquetage, par friction ou par verrouillage type baïonnette, de l'applicateur 2 sur le récipient 3.

Le manche 20, la portion de support 21 et la jupe 23 peuvent être réalisés par moulage d'un seul tenant en matière plastique.

L'élément d'application 22 peut être fixé de différentes manières sur la portion de support 21, par exemple par soudage, collage ou avec au moins un élément de fixation rapporté.

L'élément d'application 22 présente une surface extérieure 24 apte à se charger en produit P et servant à l'application.

Dans l'exemple considéré, l'élément d'application 22 comporte une mousse. En variante, l'élément d'application 22 peut comporter un feutre ou un fritté.

L'élément d'application 22 peut être configuré de manière à ce que, lorsque l'applicateur 2 est en place sur le récipient 3, l'élément d'application 22 soit reçu dans le logement 8 en étant comprimé. En variante, l'élément d'application 22 est reçu dans le logement 8 sans être comprimé.

L'applicateur 2 comporte un excitateur électrique 29 muni de deux électrodes 30 pouvant être reliées électriquement à une source électrique 32 logée dans un compartiment 33 du manche 20. Ces électrodes 30 sont par exemple fixées rigidement au manche 20.

La source électrique 32 peut comporter par exemple une pluralité de piles boutons 35 disposées en série. En variante, la source électrique peut comporter une pile sèche de 4,5 ou 9 volts, par exemple. Bien entendu, l'invention n'est pas limitée à une source électrique particulière, et l'on peut utiliser d'autres types de piles ou accumulateurs électriques ou d'intensité prédéfinie ou encore un adaptateur secteur, non représenté.

La tension délivrée par les électrodes 30 est par exemple une tension continue, mais l'excitateur électrique 29 pourrait comporter un circuit électronique permettant par exemple de délivrer un courant impulsionnel ou alternatif de tension abaissée ou augmentée par rapport à celle délivrée par la source.

L'excitateur électrique 29 peut comporter un interrupteur 37, disposé sur une face extérieure de l'applicateur 2, permettant de contrôler l'alimentation électrique des électrodes 30.

L'excitateur électrique 29 peut également comporter un témoin lumineux 38 pouvant s'allumer par exemple lorsqu'une tension est appliquée aux électrodes 30.

En variante, le témoin lumineux 38 ne peut s'allumer que lorsqu'un courant électrique d'intensité prédéfinie circule entre les électrodes 30.

En variante encore, le témoin lumineux 38 peut s'allumer par exemple d'une première couleur lorsque les électrodes 30 sont sous tension et s'allumer d'une deuxième couleur lorsqu'un courant électrique circule entre les deux électrodes 30. Le témoin lumineux peut alors être, dans ce cas, une diode électroluminescente bicolore.

Le manche 20 comporte un capot de fermeture 39 destiné à fermer le compartiment 33 et pouvant être ouvert notamment pour remplacer la source électrique 32.

Dans l'exemple considéré, les électrodes 30 sont sensiblement cylindriques et comportent chacune une portion terminale 40 noyée complètement, de manière permanente, dans l'élément d'application 22, même lorsque celui-ci est comprimé.

Bien entendu, on ne sort pas du cadre de la présente invention lorsque les électrodes 30 présentent une forme différente, les électrodes 30 pouvant être souples ou rigides, et métalliques ou non.

Les électrodes 30 peuvent en outre présenter, le cas échéant, une forme recourbée de manière à participer au maintien de l'élément d'application 22 sur la portion de support 21.

Les électrodes 30 peuvent être concentriques ou non.

Les électrodes 30 peuvent être fixées par tous moyens sur le reste du dispositif, notamment par sertissage, soudage, encliquetage, surmoulage.

La portion de support 21 peut éventuellement être métallique et constituer une électrode de l'applicateur 2.

Dans l'exemple considéré, l'élément d'application 22 est électriquement isolant à sec, étant par exemple réalisé en un matériau électriquement isolant, et le produit P est électriquement conducteur, contenant par exemple des sels minéraux.

Le dispositif d'application 1 peut s'utiliser par exemple de la manière suivante.

On suppose qu'avant la première utilisation, l'applicateur 2 est disposé sur le récipient 3 et que l'élément d'application 22 est reçu dans le logement 8.

Le chargement de l'élément d'application 22 en produit P peut être obtenu par exemple en amenant le dispositif 1 tête en bas ou en le secouant, de sorte que du produit P s'écoule à travers les orifices 11 vers l'élément d'application 22.

Le corps de récipient 4 pourrait encore comporter des parois élastiquement déformables permettant à l'utilisateur de réduire le volume du réservoir 7 afin de favoriser l'expulsion du produit P à travers les orifices 11.

Le récipient 3 pourrait également être équipé d'une pompe, non représentée, dont l'actionnement provoque l'expulsion de produit vers l'élément d'application 22.

Une fois l'élément d'application 22 chargé en produit P, l'applicateur 2 est séparé du récipient 3.

L'utilisateur peut amener l'élément d'application 22 au contact de la zone à traiter, par exemple la peau, et le produit P est déposé par exemple sous la forme d'un film.

Simultanément à l'application du produit P sur la zone à traiter, l'utilisateur peut actionner l'interrupteur 37 pour alimenter électriquement les électrodes 30.

L'élément d'application 22 étant chargé en produit P, qui est électriquement conducteur, un courant électrique peut circuler entre les deux électrodes 30 et dans la région du corps en contact avec l'élément d'application 22.

Le passage de courant sur la peau peut activer la micro-circulation sanguine notamment, ce qui peut éventuellement faciliter la pénétration d'au moins un actif contenu dans le produit vers la peau.

En variante, l'utilisateur peut utiliser l'applicateur 2 pour simplement appliquer du produit sur une zone à traiter, sans générer de courant électrique.

En variante encore, l'utilisateur peut, dans un premier temps, appliquer du produit sur la zone à traiter à l'aide de l'applicateur 2 sans générer de courant électrique, puis, dans un deuxième temps, une fois le produit appliqué, utiliser l'élément d'application 22 pour générer un courant électrique sur la peau en massant la zone traitée, par exemple.

L'utilisateur peut aussi alterner traitements avec application d'un courant électrique et traitements sans.

Le courant électrique peut aussi être appliqué de manière périodique, par exemple en manipulant l'interrupteur 37 de façon à provoquer le passage de courant électrique par intermittence.

Dans l'exemple qui vient d'être décrit, l'excitateur électrique comporte un interrupteur distinct de la source électrique.

On a représenté sur la figure 2 un applicateur 2' se différenciant de l'applicateur 2 précédemment décrit par le fait qu'il comporte un organe de rappel élastique, par exemple un ressort hélicoïdal 42, disposé dans le fond d'un compartiment 43 ouvert à son extrémité supérieure. Une source électrique 44, par exemple une pile sèche 44 de 9 volts, est disposée dans le compartiment 43, en appui sur l'organe de rappel élastique 42, de manière à être déplaçable dans le compartiment 33 entre une première position dans laquelle la source électrique 44 alimente les électrodes 30, et une deuxième position dans laquelle la source électrique 44 est déconnectée des électrodes 30.

Dans la première position, deux bornes électriques 48 de la source électrique 44 viennent en contact avec deux bornes électriques 49 disposées dans le fond du compartiment 43.

Les bornes 49 peuvent par exemple être portées par un insert 46 sur lequel sont fixées les électrodes 30.

Lors de l'application de produit P sur la peau S, l'utilisateur peut exercer une force F suffisante sur la source électrique 44 pour l'amener dans la première position et permettre le passage d'un courant électrique entre les électrodes 30.

En relâchant la source électrique 44, l'organe de rappel élastique déplace celle-ci dans la deuxième position et le courant électrique est interrompu.

Dans l'exemple illustré à la figure 2, l'élément d'application 22 est électriquement conducteur à sec, comportant par exemple une matière plastique renfermant une charge électriquement conductrice. Le produit P peut ainsi ne pas être particulièrement conducteur.

La charge conductrice peut comporter par exemple des particules électriquement conductrices 47, notamment des particules métalliques, telles que des particules de cuivre, d'argent ou d'aluminium ou des particules de graphite.

L'élément d'application 22 peut également comporter des particules magnétiques afin de cumuler les effets du courant et d'un champ magnétique.

On ne sort pas du cadre de la présente invention lorsque l'élément d'application est fixé à demeure sur le récipient.

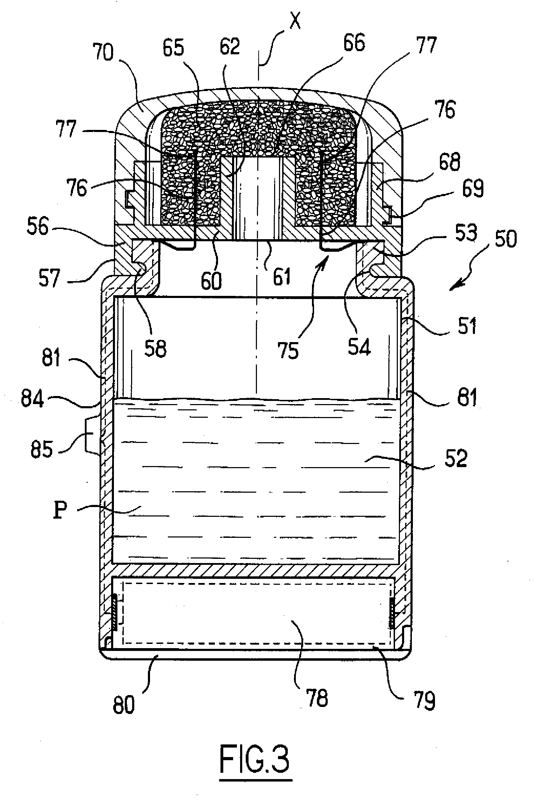

On a représenté sur la figure 3 un dispositif d'application 50 conforme à l'invention, comportant un récipient 51 formant un réservoir 52 contenant un produit P, notamment liquide.

Le récipient 51 comporte un col 53 d'axe X pourvu d'une gorge annulaire 54 permettant la fixation d'un support 56.

Ce support 56 comporte inférieurement une jupe de montage 57 avec un bourrelet annulaire 58 pouvant s'encliqueter dans la gorge 54 du récipient 51.

Le support 56 comporte une paroi transversale 60 venant en appui sur le col 53 du récipient 51, cette paroi 60 ayant un orifice central 61 débouchant dans une cheminée 62 d'axe X.

Cette cheminée 62 se raccorde sur le dessus de la paroi 60 et peut présenter une section transversale circulaire ou autre, par exemple elliptique.

Un élément d'application 65, réalisé en mousse et apte à se charger en produit P, est fixé à demeure sur le support 56, étant par exemple soudé ou collé à celui-ci.

En variante, l'élément d'application 65 est monté de manière amovible sur le support 56 de manière à pouvoir être remplacé, le cas échéant, par un autre élément d'application identique ou non présentant par exemple une forme et/ou un aspect de surface différent, l'élément d'application pouvant être sélectionné le cas échéant par l'utilisateur en fonction du produit à appliquer et/ou de la zone à traiter.

L'élément d'application 65 comporte un évidement 66 dans lequel s'engage la cheminée 62.

Du produit P peut ainsi s'écouler du réservoir 52 vers l'élément d'application 65 *via* la cheminée 62.

Le récipient 51 peut comporter des parois élastiquement déformables pour permettre à l'utilisateur de réduire le volume du réservoir 52 et provoquer l'expulsion de produit P.

Le support 56 comporte en outre, dans l'exemple illustré, une paroi cylindrique 68 d'axe X s'étendant au-dessus de la paroi transversale 60 et comportant un filetage 69 permettant la mise en place par vissage d'un capot de fermeture 70.

Dans l'exemple considéré, la mise en place du capot de fermeture 70 sur le support 56 provoque une compression de l'élément d'application 65.

En variante, l'élément d'application 65 et le capot 70 peuvent être agencés pour que, lorsque le capot 70 est en place sur le support 56, l'élément d'application 65 ne soit pas comprimé.

Le dispositif 50 comporte un excitateur électrique 75 comprenant deux électrodes 76 portées par la paroi transversale 60 du support 56. Ces électrodes 76 comportent dans l'exemple illustré chacune une portion terminale 77 entièrement noyée dans l'élément d'application 65.

Les électrodes 76 sont alimentées électriquement par une source électrique 78, par exemple une pile de 1,5 volt, représentée en pointillés sur la figure 3. Cette source électrique 78 est logée dans un compartiment 79 réalisé dans la partie inférieure du récipient 51, ce compartiment 79 pouvant être fermé à l'aide d'un capot de fermeture 80 articulé sur le récipient 51.

La source électrique 78 est reliée électriquement aux électrodes 76 par l'intermédiaire de fils électriques 81 disposés par exemple dans des gorges, non représentées, réalisées dans une paroi du récipient 51.

L'excitateur électrique 75 comporte en outre un interrupteur 85 disposé par exemple sur une paroi latérale 84 du récipient 51 et permettant de commander l'alimentation électrique des électrodes 76.

Dans l'exemple considéré, l'élément d'application 65 est électriquement isolant à sec et le produit P est électriquement conducteur.

Pour utiliser le dispositif d'application 50, l'utilisateur charge l'élément d'application 65 en produit P, par exemple en disposant le récipient 51 tête en bas.

Lorsque l'élément d'application 65 est chargé en produit, l'utilisateur l'amène au contact de la zone à traiter, par exemple la peau, et applique du produit P sur celle-ci.

L'utilisateur peut, lors de l'application, actionner l'interrupteur 85 de manière à faire circuler un courant électrique entre les électrodes 76.

L'utilisateur peut, le cas échéant, appliquer du produit sans faire circuler de courant entre les électrodes 76.

Bien entendu, l'élément d'application 65 pourrait contenir des particules électriquement conductrices afin d'accroître l'intensité du courant électrique entre les électrodes 76 et favoriser le passage du courant dans la peau.

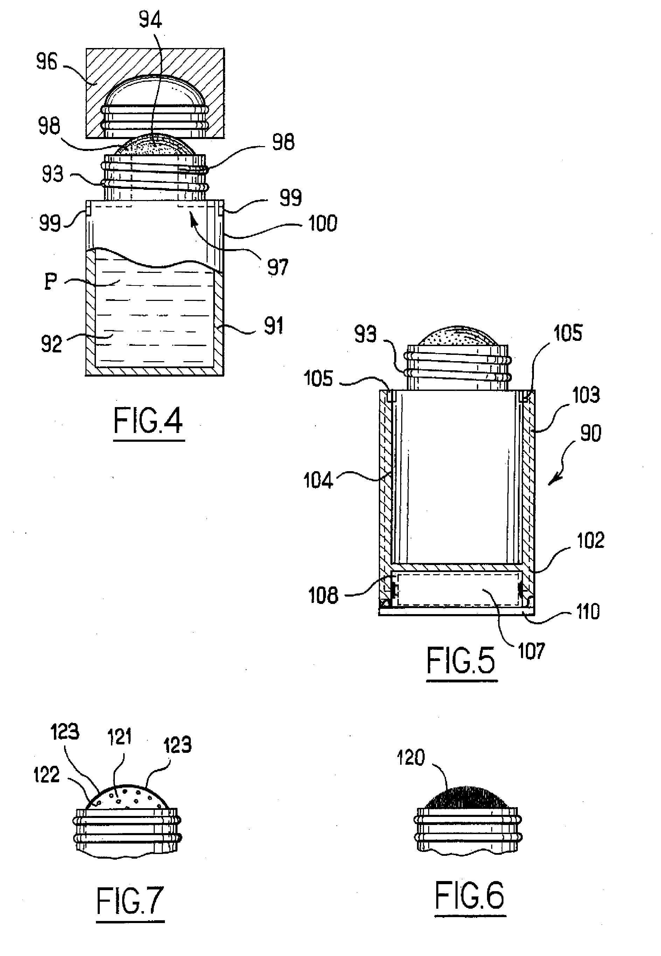

On a représenté sur les figures 4 et 5 un dispositif d'application 90 conforme à un autre exemple de mise en oeuvre de l'invention.

Le dispositif 90 comporte un récipient 91 formant un réservoir 92 pour un produit P, lequel est par exemple relativement conducteur.

Le récipient 91 comporte un col fileté 93 dans lequel est logé un élément d'application 94.

Le col fileté 93 permet la mise en place d'un capot de fermeture 96.

Le récipient 91 comporte un excitateur électrique 97 pourvu de deux électrodes 98 partiellement noyées dans l'élément d'application 94.

Ces électrodes 98 sont reliées à des contacteurs électriques 99 disposés sur une face extérieure 100 du récipient 91, par exemple à proximité du col 93.

Le dispositif d'application 90 comporte en outre un boîtier 102 pourvu d'une paroi cylindrique 103 définissant un logement 104 pour recevoir le récipient 91, comme illustré sur la figure 5.

Cette paroi 103 est pourvue sur sa surface intérieure de contacteurs électriques 105 destinés à venir au contact des contacteurs 99 du récipient 91, lorsque celui-ci est engagé dans le logement 104.

Les contacteurs 105 sont reliés, par exemple par des fils électriques, à une source électrique 107 logée dans un compartiment 108 réalisé à la partie inférieure du boîtier 102.

Ce boîtier 102 peut comporter un capot de fermeture 110 pour fermer le compartiment 108.

Le dispositif d'application 90 peut s'utiliser de la manière suivante.

Lorsque l'utilisateur souhaite simplement appliquer du produit P sur la peau, sans générer de courant électrique, il peut utiliser le récipient 91 séparément du boîtier 102.

Pour appliquer du produit sur la peau tout en générant un courant électrique, l'utilisateur met en place le récipient 91 dans le logement 104 du boîtier 102 de manière à ce que les contacteurs 99 et 105 soient reliés électriquement et qu'un courant électrique puisse circuler entre les électrodes 98.

On a illustré à la figure 6 la possibilité pour l'élément d'application de comporter une touffe 120 de poils et à la figure 7 la possibilité pour l'élément d'application de comporter une pièce 121 en élastomère par exemple, adaptée à retenir du produit par capillarité. La pièce 121 peut être réalisée en élastomère, par exemple en silicone. Au moins un orifice 122 peut déboucher sur sa surface extérieure pour l'alimenter en produit.

Des électrodes 123 peuvent également être présentes en surface, le cas échéant.

Dans toute la description, y compris les revendications, l'expression « comportant un » doit être comprise comme étant synonyme de « comportant au moins un », sauf si le contraire est spécifié.

## Revendications

1. Dispositif d'application (1 ; 50 ; 90) d'un produit, comportant :
- un élément d'application (22 ; 65 ; 94 ; 120 ; 121) ayant une surface (24) apte à se charger en produit,
- un excitateur électrique (29 ; 75 ; 97) comportant au moins deux électrodes (30 ; 76 ; 98 ; 123) en contact permanent avec l'élément d'application et permettant de faire circuler un courant électrique entre ces deux électrodes.

2. Dispositif selon la revendication précédente, **caractérisé par le fait que** l'élément d'application (22) est électriquement conducteur à sec.

3. Dispositif selon la revendication précédente, **caractérisé par le fait que** l'élément d'application (22) comporte un matériau intrinsèquement peu conducteur, notamment une matière plastique, renfermant une charge électriquement conductrice.

4. Dispositif selon la revendication précédente, **caractérisé par le fait que** la charge contient des particules (47) électriquement conductrices, notamment métalliques, choisies notamment dans la liste suivante : particules de cuivre, d'aluminium, d'argent et de graphite.

5. Dispositif selon la revendication 1, **caractérisé par le fait que** l'élément d'application (22) est électriquement isolant à sec.

6. Dispositif selon l'une quelconque des revendications précédentes, **caractérisé par le fait que** l'élément d'application est poreux.

7. Dispositif selon l'une quelconque des revendications précédentes, **caractérisé par le fait que** l'élément d'application (22 ; 65 ; 94) est élastiquement déformable.

8. Dispositif selon l'une quelconque des revendications précédentes, **caractérisé par le fait que** l'élément d'application (22 ; 65 ; 94) comporte au moins l'un des éléments choisis dans la liste suivante : une mousse, un fritté, un feutre, une touffe de poils (120), une pièce (121) en élastomère, notamment en silicone, adaptée à retenir du produit par capillarité.

9. Dispositif selon l'une quelconque des revendications précédentes, **caractérisé par le fait que** l'élément d'application est hydrophile.

10. Dispositif selon l'une quelconque des revendications précédentes, **caractérisé par le fait que** l'une au moins des électrodes (30 ; 76 ; 98) est au moins partiellement noyée dans l'élément d'application (22 ; 65 ; 94).

11. Dispositif selon l'une quelconque des revendications précédentes, **caractérisé par le fait que** l'une au moins des électrodes (30 ; 76 ; 98) ne présente aucun contact avec une surface de l'élément d'application destinée à venir au contact de la zone à traiter.

12. Dispositif selon l'une quelconque des revendications précédentes, comportant un élément de préhension (20), **caractérisé par le fait que** l'une au moins des électrodes (30) est rigidement fixée à l'élément de préhension (20).

13. Dispositif selon l'une quelconque des revendications précédentes, **caractérisé par le fait qu'**il comporte un compartiment (33 ; 79 ; 108) pour recevoir une source électrique (32 ; 78 ; 107).

14. Dispositif selon la revendication précédente, **caractérisé par le fait que** la source électrique comporte au moins une pile électrique (32 ; 44 ; 78 ; 107), un accumulateur ou un condensateur.

15. Dispositif selon l'une quelconque des revendications 1 à 13, **caractérisé par le fait que** l'excitateur électrique comporte des moyens de raccordement à un adaptateur électrique branché sur le secteur.

16. Dispositif selon l'une quelconque des revendications précédentes, **caractérisé par le fait que** la tension aux bornes des électrodes (30 ; 76 ; 98) est inférieure à 30 V, étant de préférence comprise entre 1 V et 9 V.

17. Dispositif selon l'une quelconque des revendications précédentes, **caractérisé par le fait que** l'excitateur électrique comporte un organe de réglage de la puissance électrique.

18. Dispositif selon l'une quelconque des revendications précédentes, **caractérisé par le fait que** l'excitateur électrique comporte un témoin lumineux (38) qui s'allume lorsque le courant circule entre les électrodes.

19. Dispositif selon l'une quelconque des revendications précédentes, **caractérisé par le fait qu'**il comporte un réservoir (7 ; 52 ; 92) contenant le produit (P) et agencé pour recharger l'élément d'application en produit.

20. Dispositif selon la revendication précédente, **caractérisé par le fait que** le produit (P) est électriquement conducteur.

21. Dispositif selon l'une quelconque des revendications précédentes, **caractérisé par le fait que** le produit est un produit cosmétique, notamment destiné à effectuer un traitement capillaire, ou dermatologique.

22. Dispositif selon l'une quelconque des revendications précédentes, **caractérisé par le fait qu'**il comporte, d'une part un récipient avec un réservoir de produit, et d'autre part, un applicateur (2) comprenant un élément de préhension (20) solidaire de l'élément d'application (22), l'applicateur étant agencé pour être mis en place sur le récipient de manière amovible, pour charger l'élément d'application en produit.

23. Dispositif selon la revendication précédente, **caractérisé par le fait que** l'élément de préhension (20) comporte un compartiment (33) pour loger une source électrique.

24. Dispositif selon l'une des revendications 22 et 23, **caractérisé par le fait que** le récipient (3) comporte un logement (8) pour recevoir l'élément d'application (22) lorsque l'applicateur (2) est monté sur le récipient.

25. Dispositif selon la revendication précédente, **caractérisé par le fait que** ledit logement (8) comporte une paroi de fond (10) agencée pour limiter le passage de produit du réservoir (7) vers l'élément d'application (22).

26. Dispositif selon la revendication précédente, **caractérisé par le fait que** la paroi de fond (10) comporte un ou plusieurs orifices (11).

27. Dispositif selon l'une quelconque des revendications 24 à 26, **caractérisé par le fait que**, lorsque l'élément d'application (22) est disposé dans le logement (8) du récipient en vue du stockage, l'élément d'application (22) est comprimé.

28. Dispositif selon l'une quelconque des revendications 1 à 21, comportant un récipient (51 ; 91) contenant le produit à appliquer, **caractérisé par le fait que** l'élément d'application (65 ; 94) est fixé à demeure sur le récipient.

29. Dispositif selon la revendication précédente, **caractérisé par le fait que** le récipient comporte un compartiment (79) pour loger la source électrique.

30. Dispositif selon la revendication 28, **caractérisé par le fait qu'**il comporte un boîtier (103) distinct du récipient, agencé pour loger une source électrique (107) et recevoir au moins partiellement ledit récipient (91), de manière amovible, le boîtier comportant des premiers contacteurs électriques (105) agencés pour coopérer avec des deuxièmes contacteurs électriques (99) solidaires du récipient pour alimenter électriquement les électrodes.

31. Dispositif selon l'une quelconque des revendications précédentes, **caractérisé par le fait qu'**il comporte un interrupteur (37) pour contrôler l'alimentation électrique des électrodes.

32. Dispositif selon l'une quelconque des revendications 1 à 30, comportant un élément de préhension avec un compartiment (43) pour loger une source électrique, **caractérisé par le fait que** le compartiment est pourvu d'un organe de rappel élastique (46), disposé notamment dans le fond du compartiment, agencé pour déplacer la source électrique (44) d'une première position dans laquelle la source électrique alimente les électrodes vers une deuxième position dans laquelle la source électrique est déconnectée des électrodes.

33. Dispositif selon la revendication précédente, **caractérisé par le fait que** l'organe de rappel élastique comporte un ressort (44).

34. Applicateur comportant :
- un élément de préhension (20),
- un élément d'application (22) solidaire de l'élément de préhension (2) et ayant une surface (24) apte à se charger en produit,
- un excitateur électrique (29) comportant au moins deux électrodes (30) en contact permanent avec l'élément d'application et permettant de faire circuler un courant électrique entre ces deux électrodes,
l'applicateur étant dépourvu de réservoir de produit associé à l'élément d'application, permettant de l'alimenter en produit.

35. Applicateur selon la revendication précédente, **caractérisé par le fait qu'**il comporte un compartiment pour loger une source électrique.

36. Procédé de traitement cosmétique, notamment d'une zone de la peau, comportant les étapes suivantes :
- fournir un dispositif d'application d'un produit, comportant :
- un élément d'application (22 ; 65 ; 94) ayant une surface apte à se charger en produit,
- un excitateur électrique (29 ; 75 ; 97) comportant au moins deux électrodes en contact permanent avec l'élément d'application pour faire circuler un courant électrique entre ces deux électrodes,
- charger l'élément d'application avec du produit (P),
- appliquer du produit sur la zone à traiter à l'aide de l'élément d'application.

37. Procédé selon la revendication précédente, **caractérisé par le fait qu'**il comporte l'étape suivante :
- faire circuler un courant électrique entre les deux électrodes simultanément à l'application de produit sur la zone à traiter.

38. Procédé selon la revendication 36, **caractérisé par le fait que** du produit est appliqué à l'aide de l'élément d'application sans faire circuler de courant électrique entre les électrodes.

39. Procédé selon la revendication précédente, **caractérisé par le fait qu'**il comporte l'étape suivante :
- faire circuler un courant électrique entre les électrodes sensiblement sans appliquer une quantité supplémentaire de produit, l'élément d'application étant en contact avec la zone déjà recouverte de produit.

40. Procédé selon l'une quelconque des revendications 36 à 39, l'élément d'application étant électriquement isolant à sec, le procédé étant **caractérisé par le fait qu'**il comporte les étapes suivantes :
- charger l'élément d'application avec un produit électriquement conducteur,
- faire circuler un courant électrique entre les électrodes.
